# EUROPEAN PATENT APPLICATION

(11) **EP 2 108 304 A2**
(43) Date of publication of application: **14.10.2009**
(21) Application number: 09005169.9
(22) Date of filing: 08.04.2009
(51) Int. Cl.: A61B 1/018, A61B 17/04

(54) **Medical treatment system and suturing method**

(30) Priority: 10.04.2008 US 100697
(71) Applicant: Olympus Medical Systems Corporation, Tokyo 151-0072 (JP)
(72) Inventor: Miyano, Hiromichi, Shibuya-ku Tokyo 151-0072 (JP); Yamamoto, Tetsuya, Shibuya-ku Tokyo 151-0072 (JP); Kogasaka, Takahiro, Shibuya-ku Tokyo 151-0072 (JP); Matsuno, Kiyotaka, Shibuya-ku Tokyo 151-0072 (JP); Yamatani, Ken, Shibuya-ku Tokyo 151-0072 (JP); Suzuki, Satoko, Shibuya-ku Tokyo 151-0072 (JP)
(74) Representative: von Hellfeld, Axel

(57) **Abstract**

A medical treatment system (1, 20, 40) including an endoscope (100), a treatment tool (50, 70, 71,72) that is inserted in a channel (29, 30, 81A, 81B, 81C,102, 103) of the endoscope, and a holder (2, 21, 41, 45) that holds the treatment tool in a manner that enables a user who operates the endoscope to move the treatment tool forward and backward and rotate the treatment tool, with the treatment tool having a treatment portion (51) that performs treatment on a living body; a wire (52) for operating the treatment portion whose distal end is connected to the treatment portion; a sheath portion (53) that has a first sheath (57) that is formed with a coil and in which the wire is inserted in a manner capable of moving forward and backward in the axial direction, and a second sheath (58) that is formed with a coil of a plurality of layers and provided on the outside of the first sheath; and an operating portion (55) to which the base end of the wire is connected and enables forward/backward and rotation operation of the wire; and with the holder holding the operating portion so as to be in a predetermined positional relation with respect to the endoscope.

## Description

### BACKGROUND OF THE INVENTION

### Field of the Invention

The present invention relates to a medical treatment system that includes an endoscope and an endoscope treatment tool that is inserted in the endoscope, and a suturing method that uses the endoscope treatment tool.

Priority is claimed on United State Patent Application No. 12/100,697, filed on April 10, 2008, the content of which is incorporated herein by reference.

### Description of Related Art

An endoscope treatment tool is conventionally inserted in a working channel of a soft endoscope of which the insertion portion possesses flexibility to perform various procedures in a body cavity of a patient or the like.

Among these procedures, procedures such as endoscopic suture or ligation that involve repeatedly rotating or moving the treatment tool back and forth are typically performed by a plurality of operators, with separate operators in charge of the endoscope operation and the treatment tool operation. However, in such a case, since cooperation and collaboration among the operators is not easy, and the efficiency is not always good, it would be ideal for a single operator to operate the endoscope and the treatment tool if possible.

In order for a single operator to control an endoscope and a treatment tool, it is necessary to fix the treatment tool to a forceps opening that communicates with the working channel of the endoscope. For example, Japanese Unexamined Patent Application No. 2005-58749 discloses an adapter for an endoscope that serves as a holder for such fixing purposes.

However, the holder that is disclosed in Japanese Unexamined Patent Application No. 2005-58749 does not have a mechanism that corresponds to a rotation operation, and so fixing the operating portion of a treatment tool to a forceps opening in a state that allows rotational operation is difficult.

The present invention was achieved in view of the above circumstances, and has as its object to provide a holder that can fix a treatment tool to an endoscope in a state that allows rotational operation readily.

Another object of the present invention is to provide a suturing method that can be readily performed using a treatment tool that has been inserted in an endoscope.

### SUMMARY OF THE INVENTION

The present invention is a medical treatment system that includes an endoscope, a treatment tool that is inserted in a channel of the endoscope, and a holder that holds the treatment tool in a manner that enables a user who operates the endoscope to move the treatment tool forward and backward and rotate the treatment tool; the treatment tool having a treatment portion that performs treatment on a living body; a wire for operating the treatment portion whose distal end is connected to the treatment portion; a sheath portion that has a first sheath that is formed with a coil and in which the wire is inserted in a manner capable of moving forward and backward in the axial direction, and a second sheath that is formed with a coil of a plurality of layers and provided on the outside of the first coil; and an operating portion to which the base end of the wire is connected and enables forward/backward and rotation operation of the wire; with the holder holding the operating portion so as to be in a predetermined positional relation with respect to the endoscope.

### BRIEF DESCRIPTION OF THE DRAWINGS

FIG 1 is a drawing that shows the constitution of the medical treatment system of the first embodiment of the present invention.
FIG 2 is an enlarged view showing a portion of the endoscope of the medical treatment system.
FIG. 3 shows the mounting the holder of the medical treatment system on a forceps opening of the endoscope.
FIG. 4 shows the mounting of the holder on the forceps opening.
FIG. 5 is a cross-sectional view of the holder that is mounted on the forceps opening.
FIG. 6 shows the fixing member of the holder.
FIG. 7A is an enlarged view of the holding portion of the needle holder, and FIG. 7B is an enlarged view that shows the state of the holding portion opened.
FIG 8 is a cross-sectional view that shows the main body of the needle holder, which is the treatment tool of the medical treatment system, and the operating portion.
FIG 9 is an enlarged cross-sectional view of the main body.
FIG 10 is a cross-sectional view that shows another example of the slider of the needle holder.
FIG. 11 shows the operation during use of the medical treatment system.
FIG 12 shows the operation during use of the medical treatment system.
FIG 13 shows an example of a method of approaching the target tissue.
FIG. 14 shows a step of the suturing operation using the treatment tool.
FIG 15 shows a step of the suturing operation using the treatment tool.
FIG. 16 shows a step of the suturing operation using the treatment tool.
FIG. 17 shows a step of the suturing operation using the treatment tool.
FIG. 18 shows a step of the suturing operation using the treatment tool.
FIG. 19 shows a step of the suturing operation using the treatment tool.
FIG. 20 shows a step of the suturing operation using the treatment tool.
FIG. 21 shows a step of the suturing operation using the treatment tool.
FIG. 22 shows another example of the suturing operation using the treatment tool.
FIG. 23 is a cross-sectional view that shows a holder and a treatment tool of a modification of the embodiment.
FIG. 24 shows the constitution of the medical treatment system of the second embodiment of the present invention.
FIG. 25 is a cross-sectional view of the case of the holder of the medical treatment system.
FIG. 26 shows the constitution of the medical treatment system of the third embodiment of the present invention.
FIG. 27 shows the holder in the medical treatment system of a modification of the same embodiment.
FIG. 28 shows the medical treatment system of a modification of the present invention that uses an overtube.
FIG. 29 is a cross-sectional view of the overtube.
FIG. 30 shows another example of a suture.
FIG. 31 shows the curved needle and another example of the thread.
FIG 32 shows another example of the adjustment operation of the direction of holding the curved needle.
FIG 33A and FIG 33B both show another example of the holding portion of the needle holder.

### DETAILED DESCRIPTION OF THE INVENTION

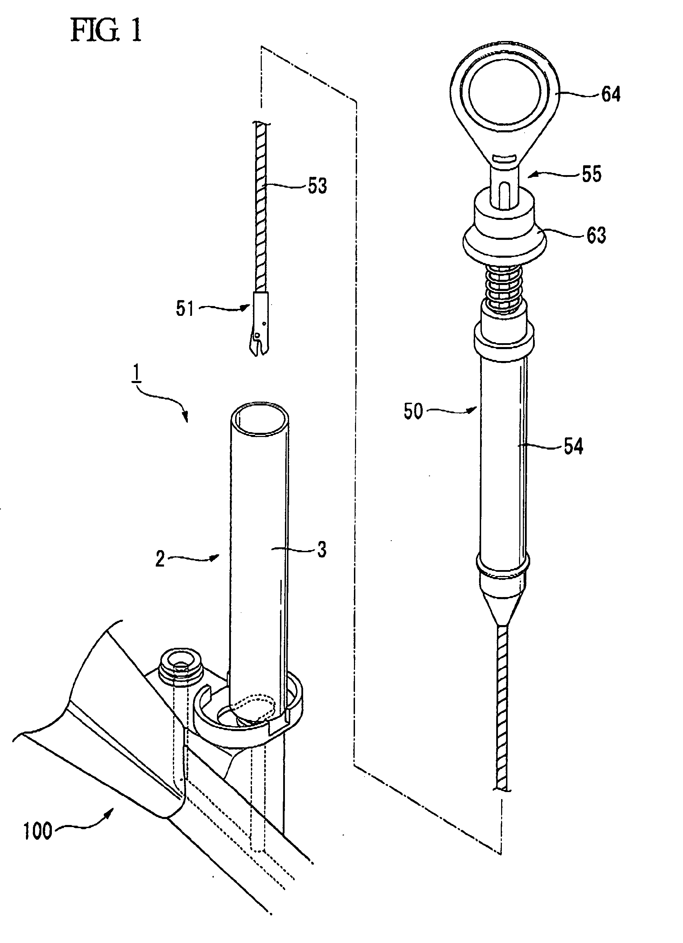

Hereinbelow, the medical treatment system of a first embodiment of the present invention shall be described with reference to FIG. 1 to FIG. 22. As shown in FIG. 1, a medical treatment system 1 of the present embodiment is constituted by an endoscope 100, a needle holder 50 that is a treatment tool that is inserted in the endoscope, and a holder 2 for fixing the needle holder 50 to the endoscope 100.

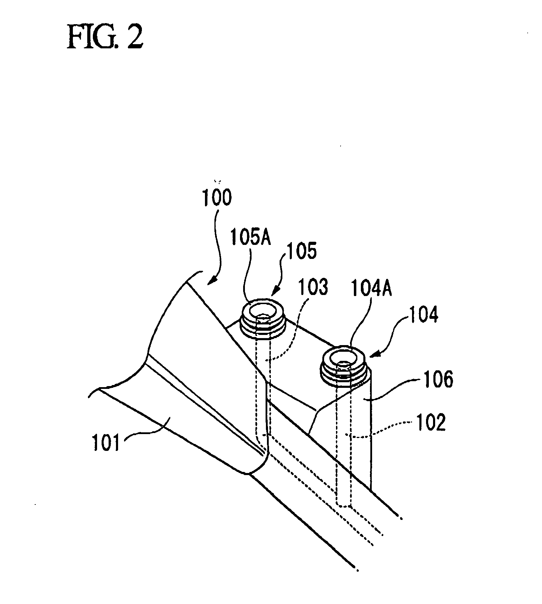

The endoscope 100 is a publicly known two-channel scope in which two channels are provided in the insertion portion that is inserted into a body cavity, but a one-channel scope may also be used. As shown in FIG 2, a first forceps opening 104 and a second forceps opening 105 that respectively communicate with a first channel 102 and a second channel 103 are provided in the vicinity of a handle 101 of the endoscope 100. Each forceps opening 104, 105 has a flange 104A, 105A, respectively, that projects from a case 106 that is attached to the handle, with the end portion of each having a large diameter.

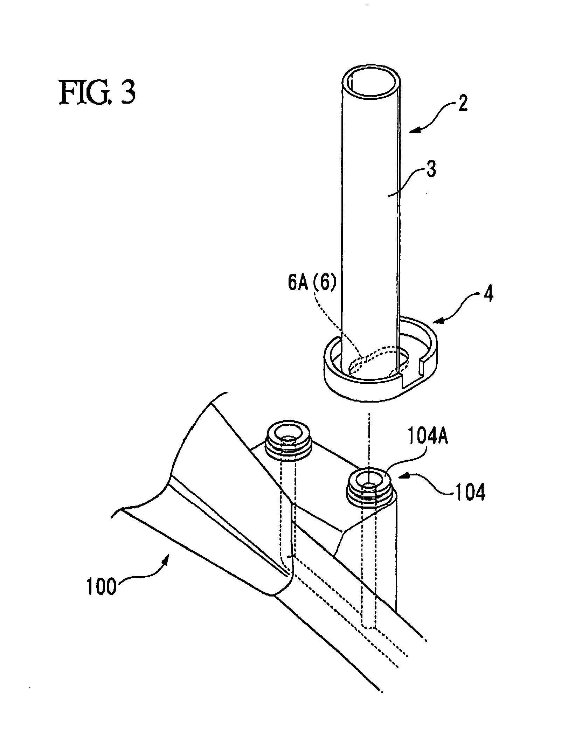

As shown in FIG. 3 to FIG. 5, the holder 2 consists of a cylindrical main body 3, a fixing member 4 that is attached to one end portion of the main body 3, and an airtight valve 5 that is attached to the lumen of the main body 3.

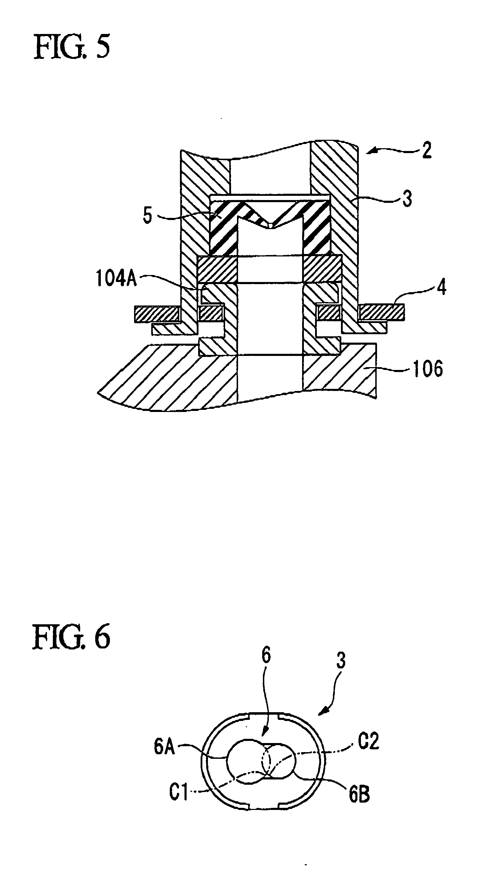

The fixing member 4 is a plate-shaped member with an approximately elliptical slide hole 6 formed in the center, as shown in FIG. 6. The dimensions of the slide hole 6 are set so that the diameter of a virtual circle C1 that is based on the outer circumference of one end portion 6A is a value not less than the diameter of the flanges 104A, 105A of the forceps openings 104, 105, and the diameter of a virtual circle C2 that is based on the outer circumference of the other end portion 6B is less than the diameter of the flanges 104A, 105A.

As shown in FIG 5, the airtight valve 5 is attached to the inside of the main body 3, and so when the needle holder 50 is inserted in the holder 2, the gas in the body cavity is prevented from leaking through the holder 2.

The needle holder 50 that is one example of a treatment tool is as shown in FIG. 1 constituted by a holding portion (treatment portion) 51 that is provided at the distal end, a wire 52 that is connected to the holding portion 51, a sheath portion 53 in which the wire 52 is inserted in a manner capable of moving back and forth, a main body 54 that is attached to a base end of the sheath portion 53, and an operation portion 55 that is attached to the main body 54.

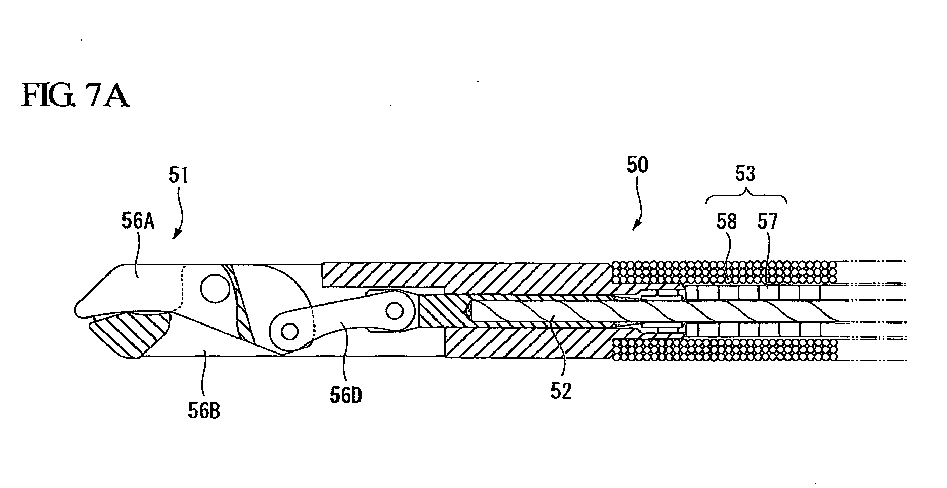

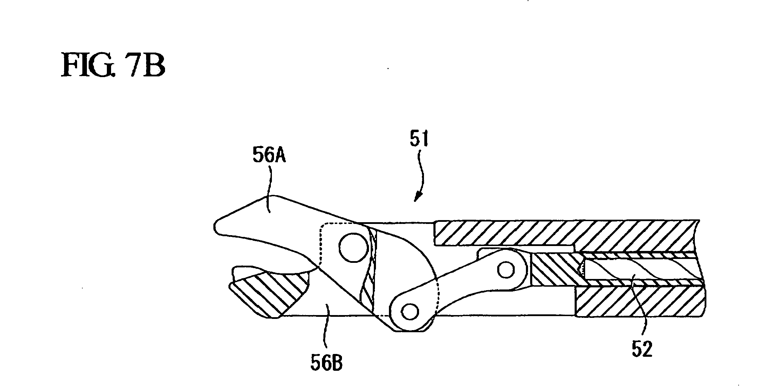

As shown up close in FIG 7A, the holding portion 51 is a publicly known constitution that has a pair of jaws 56A, 56B, with the one jaw 56A being connected to the wire 52 via a link 56D. Then, when the wire 52 moves forward to the distal end side, as shown in FIG. 7B, the jaws 56A, 56B separate and form a shape that is capable of holding a needle or the like. Because the wire 52 as described below is biased so as to move backward, the jaws 56A, 56B are biased so as to be closed other than during operation.

The sheath portion 53 in which the wire 52 is inserted is, as shown in FIG. 7A, provided with a monolayer first sheath 57 that consists of a metal flat coil being wound in a coil shape, and a second sheath S8 that consists of a metal bare wire being wound into three layers (a plurality of layers) on the outer side of the first sheath 57. The first sheath 57 is highly resistant to compression in the axial direction, and so hardly dampens the forward/backward operation of the wire 52. The second sheath 58 has high rotation compliance, and so rotating torque that is applied to the wire 52 is favorably transmitted.

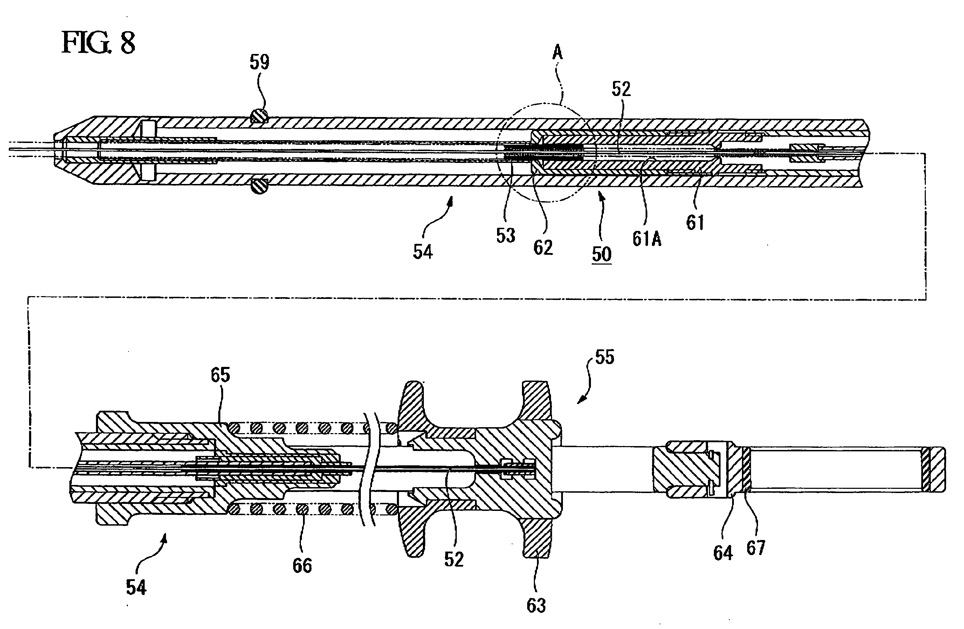

The main body 54 is an approximately cylindrical member, and as shown by its cross section in FIG. 8, the sheath portion 53 and the wire 52 are inserted therethrough. Also, a ring 59 for fixing the main body 54 to the holder 2 is attached to an arbitrary position on the outer circumference of the main body 54. As required, the main body 54 may be formed so that the cross section that intersects the axial direction is polygonal to facilitate the rotation operation described below.

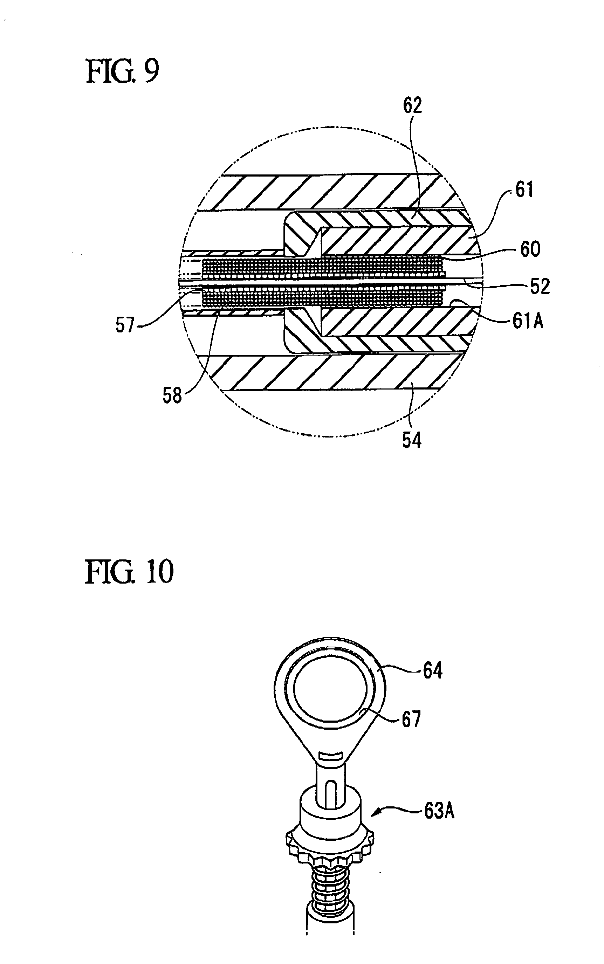

FIG 9 is an enlarged view of the portion that is included in the circle A in FIG. 8. As shown in FIG. 8 and FIG. 9, a sliding member 60 for fixing the second sheath 58 to the inside of the main body 54 is attached to the base end side of the second sheath 58. The cross-sectional shape of the sliding member 60 that intersects the axial direction is approximately cylindrical, and the sheath portion 53 is inserted along the axial line and integrally fixed by a means such as brazing.

The sliding member 60 is attached to a holding member 61 that is fixed to the inside of the main body 54 in a manner to be capable of sliding within a given range in the axial direction. A slit 61 A is formed in the axial direction in the holding member 61. The dimensions in the lengthwise direction of the sliding member 60 are approximately the same as the diameter of the holding member 61 or slightly smaller, and the dimensions in the shorter axis direction are approximately the same as the width of the slit 61A. Then, the sliding member 60 is housed in the holding member 61 so as to be sandwiched in the slit 61A of the holding member 61, and so can slide in the slit 61 A. Thereby, expansion and contraction in the axial direction by operation of the second sheath 58 is absorbed. The outer side in the circumferential direction of the sliding member 60 and the holding member 61 is covered by a cover 62, and the sliding member 60 does not deviate from the slit 61 A. The cover 62 and the holding member 61 are integrally fixed by a screw fitting. Since the sliding member 60 is housed in the slit 61A of the holding member 61, the main body 54, the sheath portion 53, and the wire 52 rotate as one unit.

The operating portion 55, as shown in FIG 8, is provided with a slider 63 and a finger hooking portion 64. The slider 63 is attached to the main body 54 to be capable of moving forward and backward, and the base end of the wire 52 is fixed. The slider 63 has an approximately cylindrical shape, but in order to facilitate operation, unevenness may be provided on the outer circumferential surface similarly to the slider 63A of the modification shown in FIG. 10.

A spring 66 is attached between the slider 63 and a large diameter portion 65 that is provided in the main body 54 to the front of the slider 63, and when the slider 63 is not operated, the slider 63 is always biased so as to separate with the large diameter portion 65 by at least a predetermined distance. That is, it is biased so that the wire 52 is pulled to the base end side. Thereby, the jaws 56A, 56B of the holding portion 51 are biased to be closed when the slider 63 is not operated.

The finger hooking portion 64 is an annular member and is attached to the base end of the main body 54. A cushion 67 made of rubber or the like is attached to the inner circumference of the finger hooking portion 64, so that even when operated over a long period the finger or the like will not become sore. Note that the finger hooking portion 64 is attached to the main body 54 so as to freely rotate about the axial line, but may also be attached so as not to rotate.

Also, the operation portion 55 may be one that is provided with a handle of a publicly known so-called gun grip type or inline type similarly to a typical needle holder.

The operation during use of the endoscope medical treatment system that is constituted as described above shall be described using as an example the case of performing suturing of a target tissue using the needle holder 50.

First, the user attaches the holder 2 to a forceps opening of the endoscope 100. The holder 2 may be attached to either forceps opening, but here the example of attaching it to the forceps opening 104 shall be described.

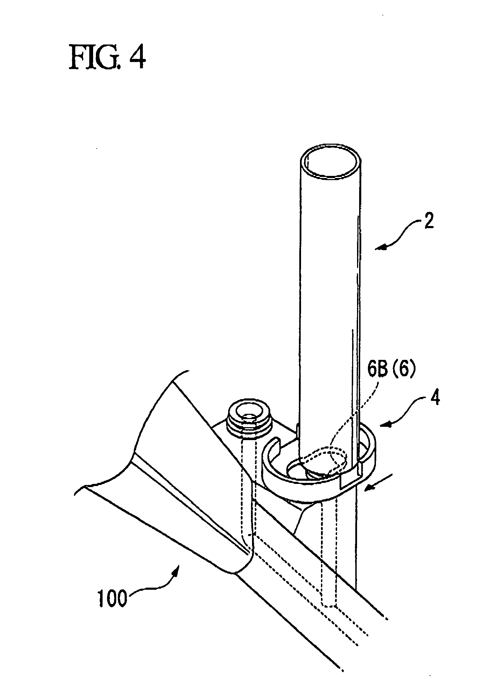

As shown in FIG 3, after passing the flange 104A of the forceps opening 104 through the end portion 6A side of the slide hole 6 of the fixing member 4, by sliding the fixing member 4 as shown in FIG. 4, the virtual circle C2 in the end portion 6B of the slide hole 6 moves to become approximately coaxial with the forceps opening 104. Thereby, the forceps opening 104 does not slip out from the slide hole 6, and the holder 2 is fixed to the forceps opening 104.

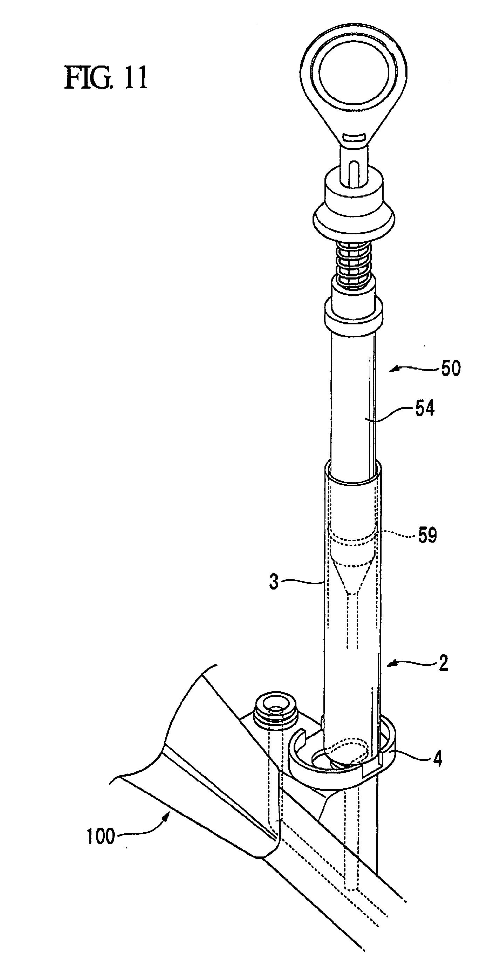

Next, as shown in FIG. 1, the user inserts the needle holder 50 from the holding portion 51 side into the main body 3 of the holder 2. Then, the holding portion 51 is projected from the distal end of the endoscope 100. When the holding portion 51 is slightly projected from the distal end of the endoscope 100, the needle holder 50 is fixed to the holder 2 by frictional force as shown in FIG 11 by the ring 59 that is attached to the main body 54 of the needle holder 50.

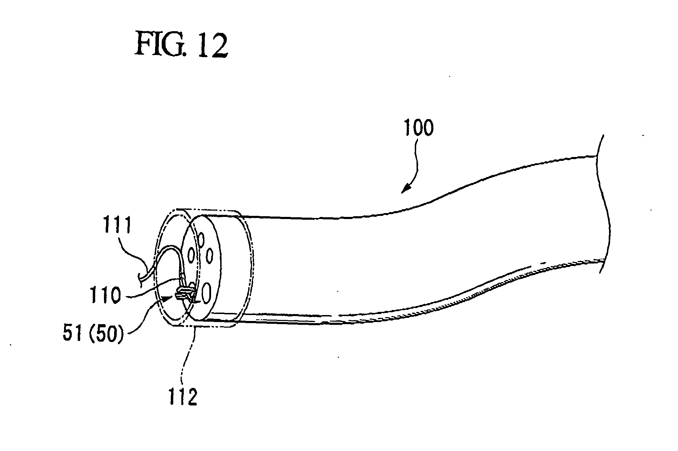

Next, the user opens the holding portion 51 by moving the slider 63 forward, and by placing a curved needle 110 with a thread 111 attached between the jaws 56A, 56B and returning the slider 63 to its original position, the curved needle 110 is made to be grasped by the holding portion 51. Then, as shown in FIG. 12, a cylindrical cap 112 having an inner diameter that is approximately the same as the outer diameter of the distal end of the endoscope 100 is mounted on the distal end of the endoscope 100, and the endoscope 100 and the needle holder 50 are inserted into a body cavity of the patient. Note that if the cap 112 is formed with a transparent material, the visibility of the surrounding tissue increases, thereby facilitating the procedure following insertion. Also, an overtube or the like may be used instead of the cap 112.

When there is a desire to change the direction in which the holding portion holds the curved needle 110 during insertion of the endoscope 100, by slightly retracting the needle holder 50 with respect to the endoscope 100, the curved needle 100 is pushed against the end face at the distal end of the endoscope 100. When this happens, the curved needle 110 rotates about the axial line, and it is possible to adjust the direction. Also, since the cap 112 is mounted on the distal end of the endoscope, the curved needle 110 is prevented from coming into contact with tissue that is not the treatment target during insertion.

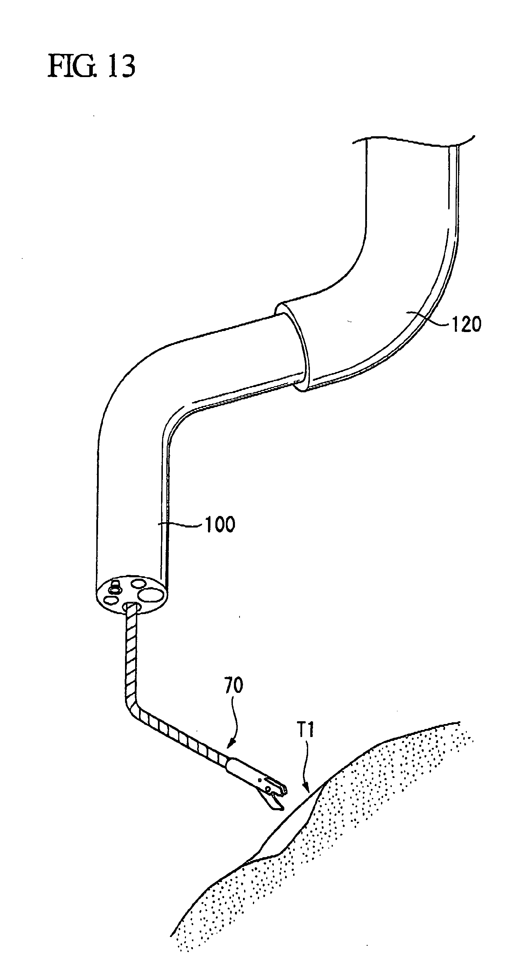

The user moves the endoscope 100 forward to move it to the tissue of the treatment target. At this time, when tissue T1 that is the treatment target is at a location that is hard to reach, as shown in FIG. 13, an overtube 120 that is capable of performing a bending operation and a treatment tool 70 that is capable of performing a bending operation are used, and by respectively bending the overtube 120, the endoscope 100, and the needle holder 70, it can be moved to the target tissue T1.

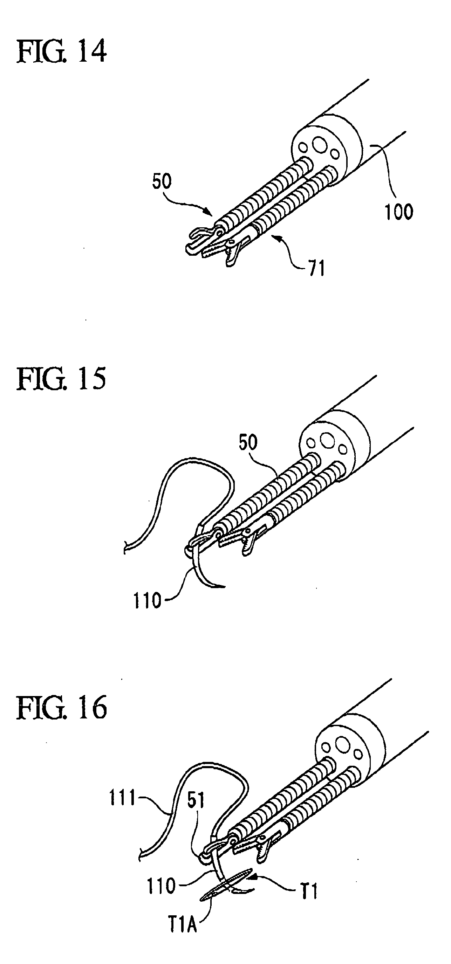

When the distal end of the endoscope 100 reaches the vicinity of the target tissue T1, the user commences the suture procedure. First, the user, as a preparation for the suture procedure, inserts a holding forceps 71 that is a second treatment tool in the forceps opening 105, and as shown in FIG 14 causes it to project from the distal end of the endoscope 100. At this time, another holder 2A with the same construction as the holder 2 may be mounted on the forceps opening 105 as required.

The suturing method shall now be described.

First the user inserts the curved needle 110 that is grasped by the needle holder 50 as shown in FIG. 15 into the periphery of a wound portion T1A that is the suture target at the target tissue T1 while rotating the holding portion 51 about the axial line as shown in FIG. 16, and extracts the curved needle 110 from a region that opposes the insertion region so as to sandwich the wound portion T1A. The thread 111 is thus applied to the wound portion T1A. This work is performed a predetermined number of times in accordance with the length of the wound portion T1A, and the thread 111 is disposed so as to be able to suture the wound portion T1A. Note, FIG. 16 to FIG. 21 show the state of the thread 111 applied to the wound portion T1A once in order facilitate visualization of the work to create a knot K described below.

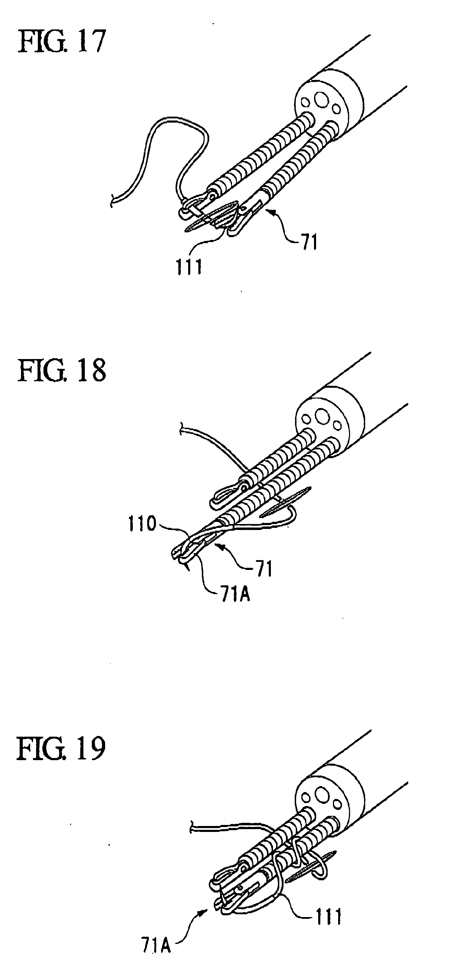

When the placement of the thread 111 is complete, the thread 111 is tied and the knot K is made. The user, as shown in FIG. 17, passes the curved needle 110 that has been extracted over to the holding forceps 71, and after moving the holding forceps 71 forward as shown in FIG. 18, rotates a holding portion 71A of the holding forceps 71 about the axial line to, as shown in FIG. 19, wind the thread 111 around the holding forceps 71. It does not matter which direction is the direction of rotation, but the direction of rotation at this time is defined as the first rotation direction.

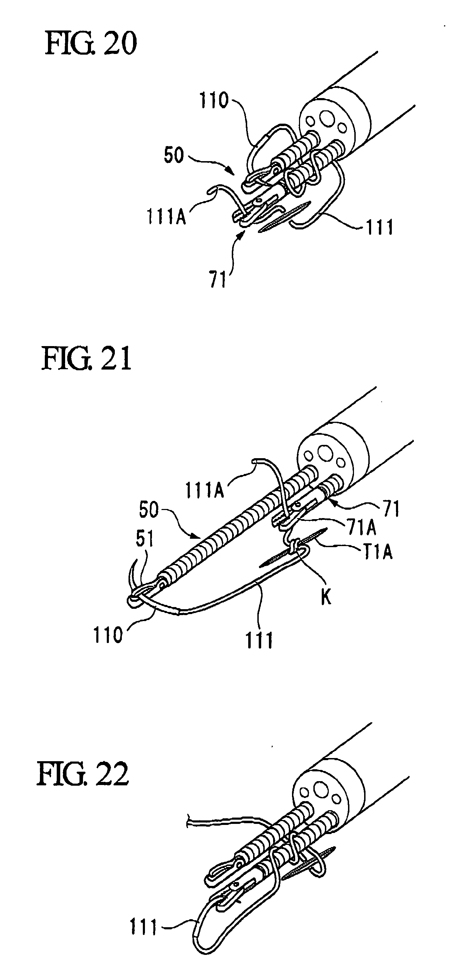

Next, the user as shown in FIG 20 passes the curved needle 110 back to the needle holder 50, and holds an end portion 111 A of the thread 111 on the opposite side of the curved needle 110 with the holding forceps 71. Then, as shown in FIG 21, while positioning the curved needle 110 ahead of the end portion 111 A of the thread 111, when the holding portion 71A of the holding forceps 71 and the holding portion 51 of the needle holder 50 are operated so as to mutually separate, the end portion 111A passes through a loop of the thread 111 that is formed by the holding forceps 71, and thus the knot K is formed in the thread 111. The knot K is a so-called single knot, and is easily loosened. Therefore, by performing a single knot with the abovementioned operation one more time, the thread 111 is knotted to securely suture the wound portion T1A.

At this time, when the second knot is made by rotating the holding forceps 71 in the first rotation direction described above again, a granny knot (first knot) results. On the other hand, when the second knot is made by rotating the holding forceps 71 in a second rotation direction that is the reverse of the first rotation direction, a square knot (second knot) results that is stronger and more secure than a granny knot. Moreover, when the above-described square knot is performed after winding of the thread 111 on the holding forceps, a surgical knot (third knot) results that is even more secure than the square knot. Whichever knot is to be formed is suitably decided based on the location of the wound portion T1A, the method of applying tension to the thread 111, and the like..

Normally, forming a knot in the thread using a treatment tool is not easy, but by operating the two treatment tools as described above, it is possible to perform suturing and ligation by readily forming knots. Note, in the abovedescribed example, the example of using the needle holder 50 and the holding forceps 71 as two treatment tools was described, but as long as the curved needle 110 can be securely grasped, the combination of the treatment tools is not particularly limited. Also, in the abovedescribed operation, the curved needle 110 may be grasped so that the distal end thereof is positioned behind the base end thereof that is connected to the thread 111 as shown in FIG. 22.

According to the endoscope medical treatment system of the present embodiment, since the operation portion 55 of the needle holder 50 is held in the forceps opening 104 of the endoscope 100 in the state of rotation operation being easy by the holder 2 having the cylindrical main body 3, a single user can readily perform operation of the endoscope 100 and operation of the needle holder 50 that is a treatment tool by himself.

Also, since the ring 59 is attached to the main body 54 of the needle holder 50, it is possible to temporarily fix the main body 54 to an arbitrary position with respect to the holder 2 by the frictional force that is produced between the ring 59 and the inner surface of the main body 3 of the holder 2, and possible to maintain the projected state of the holding portion 51 in the desired state. Also, since the ring 59 blocks the gap between the holder 2 and the needle holder 50, it is possible to more reliably prevent gas in the body cavity from leaking to the outside via the holder 2.

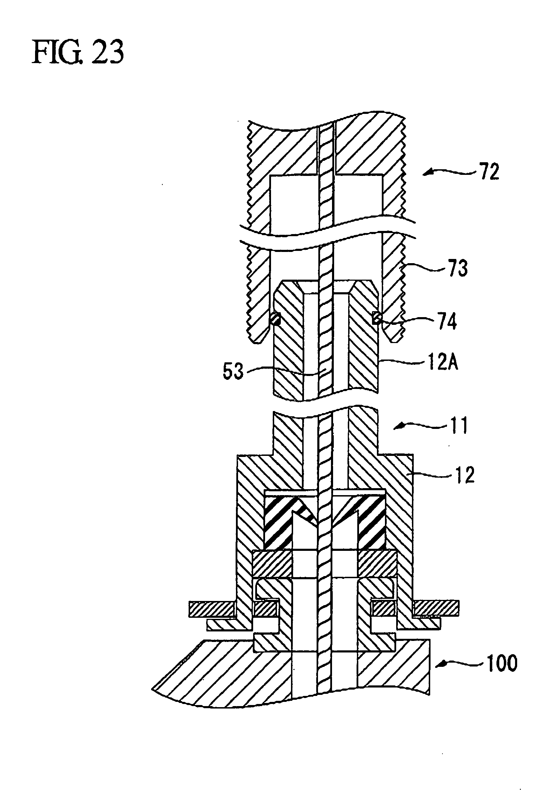

In the abovementioned embodiment, the example was described of the operation portion 55 of the needle holder 50 being inserted in the cylindrical main body 3 of the holder. However, instead of this, similarly to the modification shown in FIG. 23, a small diameter portion 12A may be provided on a main body 12 of a holder 11, so that a needle holder 72 may be held on the endoscope 100 in a rotatable manner by the small diameter portion 12A moving into a main body 73 of the needle holder 72.

By doing so, since it is possible to perform a rotation operation of the needle holder 72 by touching the distal end side of the main body 73, the hand of the user that operates the needle holder 72 is hindered from separating from the trunk, and so it is possible to more readily perform operation.

Also, since only the sheath portion 53 of the needle holder 72 is passed through the lumen of the holder 11, by making the lumen small, it is possible to stabilize the operation by preventing the sheath portion 53 from unnecessarily moving in the holder 11.

Note that in FIG 23, a ring 74 that has the same function as the ring 59 is attached to the small diameter portion 12A of the holder 11, but the ring 74 may also be attached to the lumen side of the main body 73 of the needle holder 72.

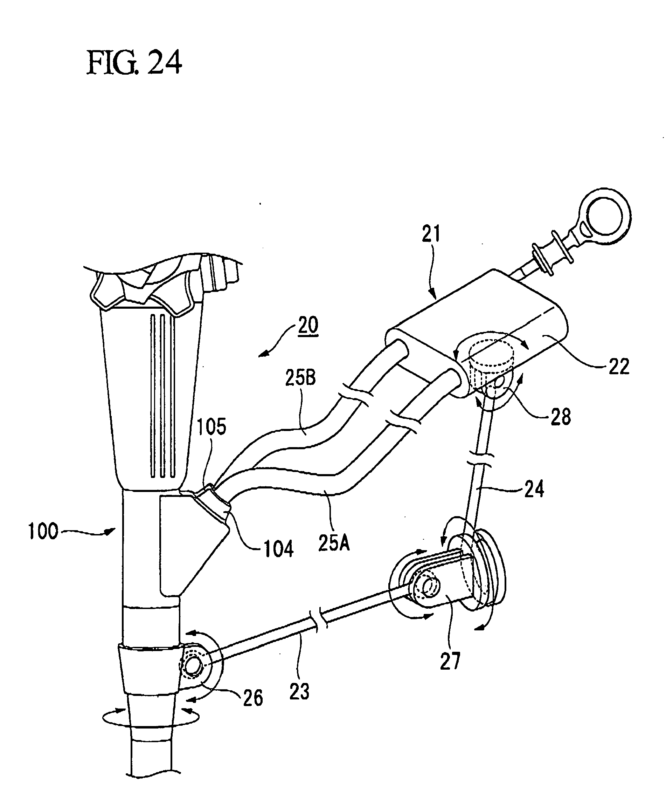

A second embodiment of the present invention shall be described with reference to FIG 24 to FIG. 26. An endoscope medical treatment system 20 of the present invention differs from the abovementioned endoscope medical treatment system on the point of being able to change the position of the holder.

Note that in the following description, constitutions that are common with the abovementioned first embodiment shall be designated by the same reference numbers, and descriptions thereof shall be omitted.

FIG. 24 shows the constitution of the endoscope medical treatment system 20 of the present embodiment. A holder 21 has a case 22 in which a treatment tool is inserted, and is held on the endoscope 100 via a first link 23 and a second link 24. The forceps openings 104, 105 of the endoscope 100 are communicated and connected with a treatment tool channel (described below) in the case 22 by respective tubes 25A and 25B.

As shown in FIG. 24, one end portion of the first link 23 is connected in a freely rotatable manner to a first joint 26 that is attached to the endoscope 100, and the other end portion is connected in a freely rotatable manner to a second joint 27. Also, one end portion of the second link 24 is connected in a freely rotatable manner to a second joint 27, and the other end portion is connected in a freely rotatable manner to a third joint 28 that is attached below the case 22.

At the joints 26, 27, 28, it is possible to hold the first link 23 and the second link 24 in the desired positional relation. Moreover, since the first joint 26 and the third joint 28 can be attached to the endoscope 100 and the case 22, respectively, in a freely rotatable manner, the user can change the positional relation of the endoscope 100 and the case 22 of the holder 21 to an arbitrary state by suitably adjusting an adjustment portion consisting of an aggregate of these mechanisms.

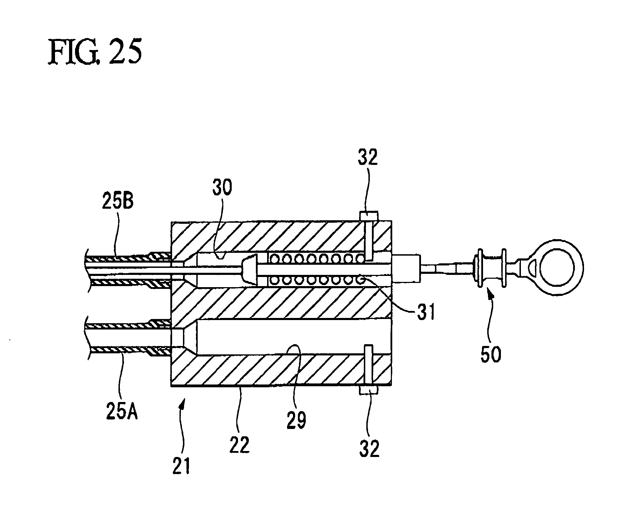

FIG. 25 is a cross-sectional drawing of the case 22. In the interior of the case, two treatment tool channels, namely, a first treatment tool channel 29 and a second treatment tool channel 30 that respectively communicate with the forceps openings 104 and 105 via the tubes 25A and 25B, are formed. Various types of treatment tools, such as the needle holder 50, are inserted in the treatment tool channels 29, 30, and are held so as to readily perform the operations of moving forward/backward and rotating.
Bearings 31 may be provided or a publicly known linear bush (not illustrated) or the like may be attached as necessary on the inner surface of the treatment tool channels 29, 30 so that a treatment tool that is inserted can smoothly move forward and backward. Also, in order to prevent an inserted treatment tool from falling out, a stopper 32 or the like that projects into the lumen of the treatment tool channels 29, 30 to engage with the main body or the like of the treatment tool may be provided.

In this endoscope medical treatment system 20 of the present invention as well, a single user can readily perform a desired procedure by operating both of the treatment tools that are held in the endoscope 100 and the holder 21.

Also, since the holder 21 is held in the endoscope 100 by the links 23 and 24 and the joints 26, 27, and 28, the user can perform a procedure in an environment where it is easier to perform operations by moving the holder 21 to a position where the user can readily perform operations.

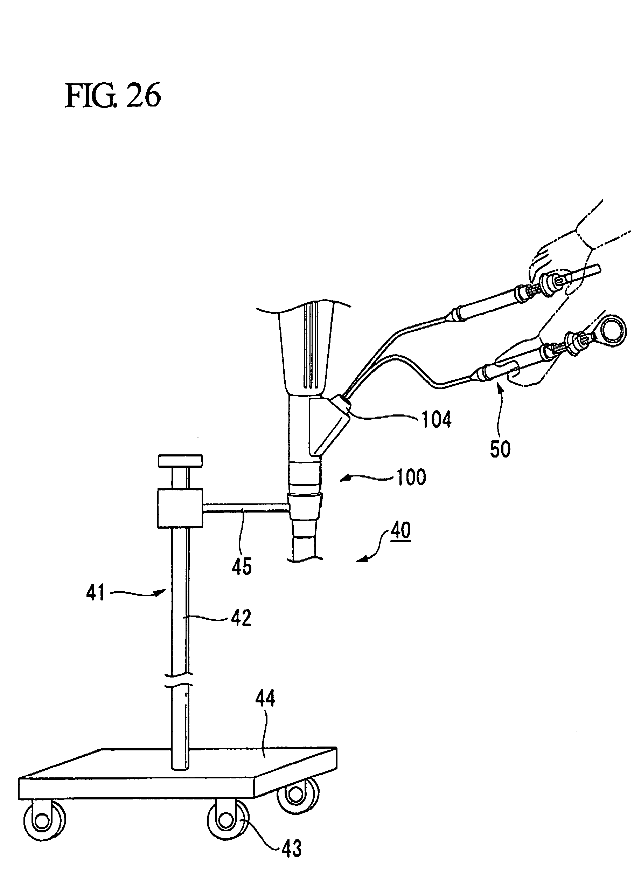

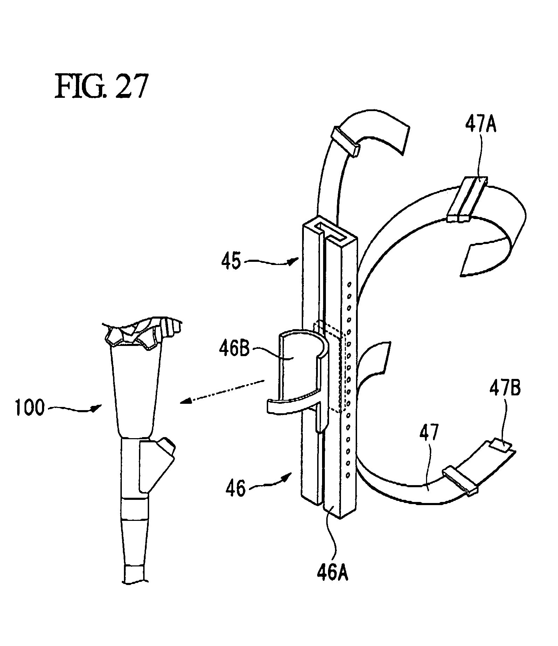

Next, a third embodiment of the present invention shall be described with reference to FIG. 26 and FIG. 27. The endoscope medical treatment system of the present embodiment differs from the abovementioned endoscope medical treatment system on the point of the user not required to hold the endoscope by hand.

FIG 26 shows the constitution of an endoscope medical treatment system 40 of the present embodiment. As shown in FIG. 26, a holder 41 that the endoscope 100 is fixed to has a column support 42 to which the endoscope 100 is attached and a caster 43, and is provided with a base 44 to which the column support 42 is attached. The caster 43 is a publicly known mechanism, and is constituted to be switchable between a rotatable state and a non-rotatable state. Also, the height of holding the endoscope 100 on the column support 42 can be adjusted by sliding a holding member 45 with respect to the column support 42.

The movement during use of the endoscope medical treatment system 40 that is constituted as mentioned above shall be described.

First, the endoscope 100 and a treatment tool such as the needle holder 50 are inserted into a body cavity of the patient with the same operation as the first embodiment. When the distal end of the endoscope 100 has moved to a preferred position in the body cavity, the user fixes the endoscope 100 to the column support 42 of the holder 41.

Then, the user removes his hand from the endoscope 100 and performs various procedures by operating the treatment tool that has been inserted in the channel of the endoscope 100. During the procedures, fine adjustment may be performed by moving the holder 41 as required. Note that FIG. 26 shows the state of two treatment tools inserted in the endoscope 100, and the user operating these treatment tools with both hands, but the number and combination of treatment tools is not particularly limited.

According to the endoscope medical treatment system 40 of the present embodiment, since it is not necessary for the user to hold the endoscope 100 in one hand, the user can focus on the operation of the treatment tool and so can more precisely execute the procedure. Also, it is possible to use in combination with the endoscope medical treatment system 20.

In the present embodiment, the example was described of the endoscope 100 being held on a holder 41 that has the column support 42 and the base 44, but the aspect of the holder that holds the endoscope 100 is not particularly limited. Hereinbelow is shown an example.

FIG. 27 is a drawing that shows an example of a holder in a modification of the present embodiment. The holder 45 has a main body 46 to which the endoscope 100 is fixed and a belt 47 that is attached to the main body 46.

The main body 46 consists of a rail 46A and a sliding body 46B that is attached to the rail 46A in a freely slidable manner. The sliding body 46B can be fixed to an arbitrary position of the rail 46A by a pin or the like that is not illustrated. The belt 47 has various publicly known mechanisms such as an adjuster 47A, a buckle (not illustrated), and a metal fitting 47B that is inserted and fixed in the buckle, and is constituted so as to fix the main body 46 to the body of the user.

In the case of using an endoscope medical treatment system that is provided with the holder 45, the user fixes the main body 46 to the trunk by the belt 47 in advance. After operating the endoscope 100 so that distal end thereof is moved to a predetermined position in a body cavity, the endoscope 100 is fixed to the sliding body 46B of the main body 46.

Since there is no need for the user to hold the endoscope 100 that is held in the holder 45 by hand, the user operates the treatment tool that has been inserted in the endoscope 100 with both hands to perform the desired procedure on the target tissue.

Even in this modification, there is no need for the user to hold the endoscope 100 by hand, so the user can focus on the operation of the treatment tool. In addition, by the user twisting his trunk or moving his upper body, it is possible to move the endoscope 100 in a given range. Therefore, it is possible to perform fine adjustment of the endoscope 100 to some extent while focusing on the operation of the treatment tool. Accordingly, the user is able to more favorably perform a procedure. Also, it is possible to use in combination with the endoscope medical treatment system 1.

While preferred embodiments of the invention have been described and illustrated above, it should be understood that these are exemplary of the invention and are not to be considered as limiting. Additions, omissions, substitutions, and other modifications can be made without departing from the spirit or scope of the present invention.

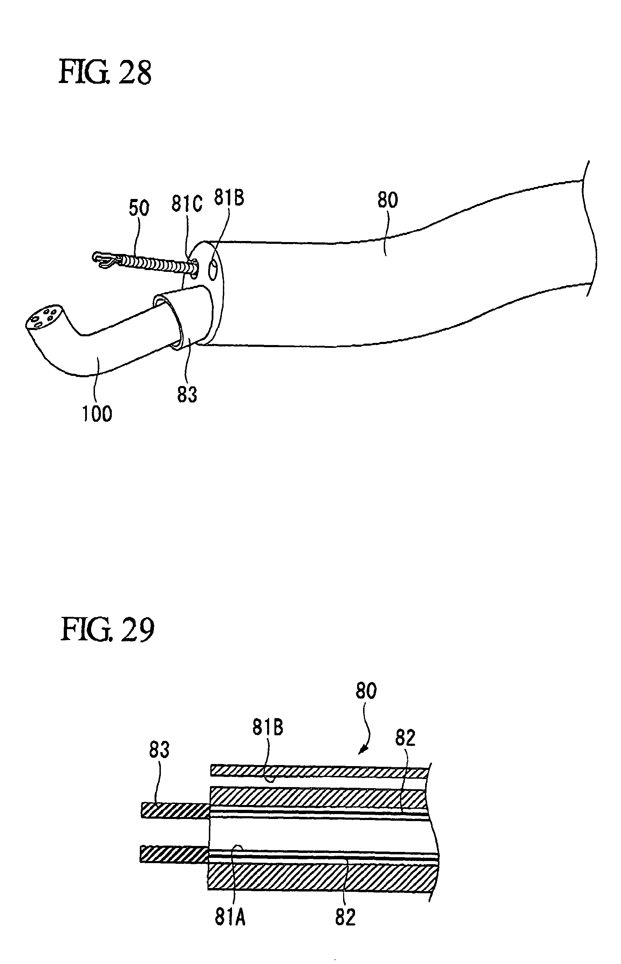

For example, in each of the embodiments described above, an endoscope medical treatment system was described in which a treatment tool was inserted in the channel of an endoscope. However, instead of this, a procedure may be performed by inserting an endoscope 100 and a treatment tool (FIG. 28 shows the example of a needle holder 50) in the channels of an overtube 80 that has the plurality of channels 81A, 81B, 81C as shown in FIG. 28 and FIG. 29. By doing so, because of the fact it is possible to separately move the endoscope 100 and the treatment tool forward and backward, during the operation of forming the aforementioned knot K, in the case of spacing out the treatment tool, it is possible to hinder the occurrence of situations such as the treatment tool leaving the field of view of the endoscope 100 as a result of the endoscope 100 moving backward in tandem.

Note that in this case, it is possible to perform procedures and the like mostly similarly to the abovementioned embodiments by a holder being provided at the base end side of the overtube 80, instead of the endoscope 100, and being attached to forceps openings and the like not illustrated that communicate with the channels 81A, 81B, 81C, and an operating portion not illustrated that is provided at the base end side of the overtube 80 being fixed to the holder.

Also, as shown in FIG. 28 and FIG. 29, by providing a curving portion 83 that enables a curving operation by wires 82 in the channel 81A through which the endoscope 100 is inserted, it is possible to look at the distal end of the treatment tool from various angles. Therefore, more procedures are facilitated, which is preferable.

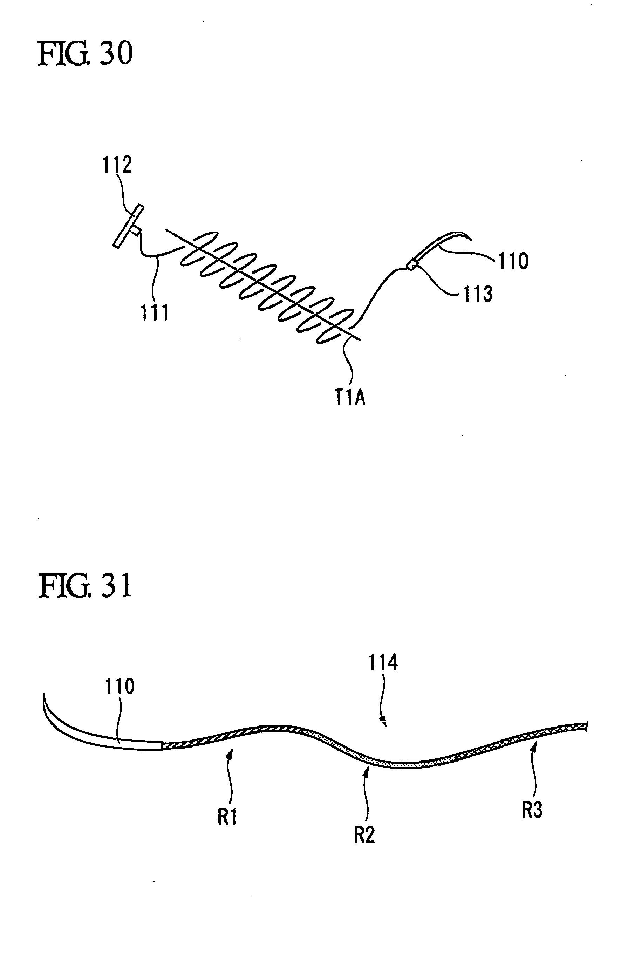

Also, in the abovementioned embodiments, the example was described of suturing or ligating by attaching the thread 111 to a wound portion T1A by the curved needle 110 with the thread 111 attached, and then forming a knot. However, instead of this, as illustrated in the modification shown in FIG. 30, suturing may also be performed by attaching an anchor 112 to one end of the thread 111, and then locking the thread 111 that has been stitched around the wound portion T1A to the wound portion T1A with a caulking member 113 or the like. By doing so, because of the fact that both ends of the thread 111 are locked to the wound portion T1A by the anchor 112 and the caulking member 113, there is no need to form a knot, and procedures such as suturing and the like can be readily performed. Note that in the present modification, when the wound portion T1A has been closed using the thread 111, the curved needle 110 is pulled out from the body cavity with the thread 111 attached. After removing the curved needle 110 and attaching the caulking member 113, the thread 111 and the caulking member 113 are returned to the body cavity, and after moving the caulking member 113 up to the vicinity of the wound portion T1A, it may be fixed by being crimped.

Also, the thread that is attached to the curved needle may be one that has regions R1, R2, R3 with different external appearances at predetermined lengths like the thread 114 as shown in FIG. 31. By doing so, even in an endoscope image in which the depth perception is insufficient, the user can carefully recognize what region of the thread 114 is being operated, and so can smoothly carry out the procedure. Note that the appearance of the regions R1 R2, R3 may be differentiated by color, or may be differentiated by pattern. Moreover, the number of regions to be set, the length of each region, and the like may be suitably set in accordance with the applicable procedure and the like.

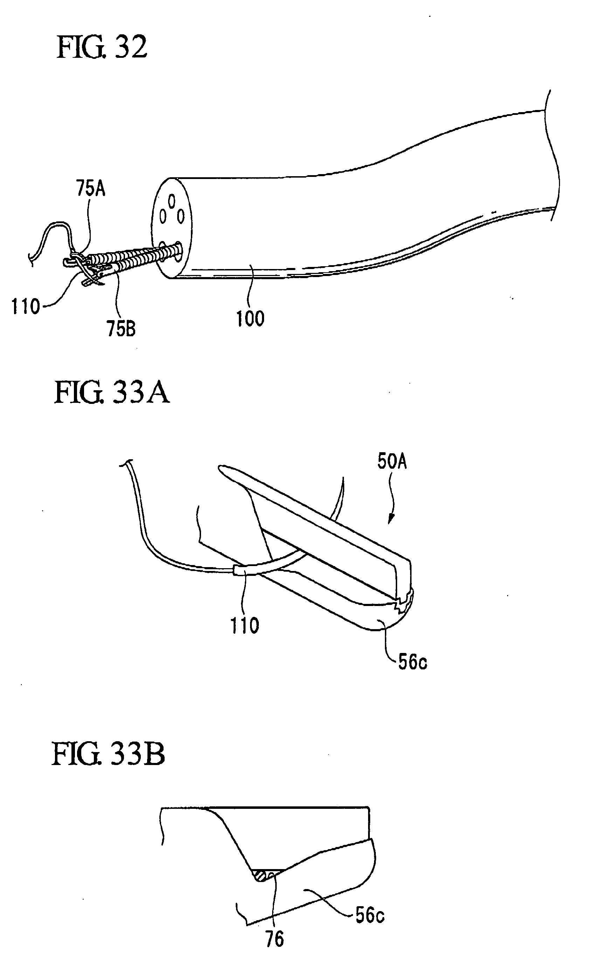

Moreover, in each of the abovementioned embodiments, the example was described of adjusting the direction in which the treatment tool holds the curved needle by causing the curved needle to abut the distal end of the endoscope. However, instead of this, it is possible to insert two treatment tools in channels of the endoscope 100 and adjust the curved needle 110 that is held by one treatment tool 75A by causing it to abut the holding portion of another treatment tool 75B as shown in FIG. 32. Also, as shown in FIG. 33A and FIG. 33B, it is possible to provide a recess portion 76 that corresponds to the curve shape of the curved needle 110 in one jaw 56C of the needle holder 50A. By doing so, the curved needle 110 that is grasped may be held in a favorable direction automatically by fitting in the recess portion 76.

Accordingly, the invention is not to be considered as being limited by the foregoing description, and is only limited by the scope of the appended claims.

## Claims

1. A medical treatment system (1, 20, 40) comprising an endoscope (100), a treatment tool (50, 70, 71, 72) that is inserted in a channel (29, 30, 81A, 81B, 81C, 102, 103) of the endoscope, and a holder (2, 21, 41, 45) that holds the treatment tool in a manner that enables a user who operates the endoscope to move the treatment tool forward and backward and rotate the treatment tool;
wherein the treatment tool has:
a treatment portion (51) that performs treatment on a living body;
a wire (52) for operating the treatment portion whose distal end is connected to the treatment portion;
a sheath portion (53) that has a first sheath (57) that is formed with a coil and in which the wire is inserted in a manner capable of moving forward and backward in the axial direction, and a second sheath (58) that is formed with a coil of a plurality of layers and provided on the outside of the first coil; and
an operating portion (55) to which the base end of the wire is connected and enables forward/backward and rotation operation of the wire;
and the holder holds the operating portion so as to be in a predetermined positional relation with respect to the endoscope.

2. The medical treatment system according to claim 1, wherein the holder is capable of being attached in a freely detachable manner to a forceps opening (104,105) that communicates with the channel of the endoscope.

3. The medical treatment system according to claim 1, wherein the holder has an adjustment portion that is capable of holding the operating portion in a plurality of different positional relations with respect to the endoscope.

4. The medical treatment system according to claim 1, wherein the treatment portion has a pair of jaws (56A, 56B) for holding a curved needle (110).

5. A method of suturing tissue (T1A) that uses a thread (111) with a needle (110) attached to a first end portion (111A); and a first treatment tool (50) and a second treatment tool (71) that are capable of holding the thread and the needle, consisting of:
a first step that holds the needle with the first treatment tool;
a second step that inserts the needle into the tissue and pulls it out by operating the first treatment tool, and places the thread in the tissue;
a third step that forms a loop by rotating the second tool about the axial line to wind the thread at least one time on the second treatment tool while holding the pulled out needle with the second treatment tool;
a fourth step that passes the needle to the first treatment tool after the third step;
a fifth step that holds a second end portion of the thread at which the needle is not attached with the second treatment tool after the fourth step; and
a sixth step that forms a knot (K) by moving the first treatment tool forward and the second treatment tool backward so as to separate the needle and the second end portion of the thread while causing the needle to be positioned ahead of the second end portion of the thread and passing the second end portion through the loop.

6. A method of suturing tissue (T1A) using a thread (111) with a needle (110) attached to a first end portion (111A), and a first treatment tool (50) and a second treatment tool (70) that are capable of holding the thread and the needle;
including a first knot forming step that forms a first knot by the suturing method according to claim 5 and a second knot forming step that forms a second knot by the suturing method according to claim 5.

7. The method of suturing tissue according to claim 6,
wherein in the third step of the second knot forming step, the second treatment tool is rotated in the opposite direction to the first knot forming step.

8. The method of suturing tissue according to claim 7,
wherein in the third step of the first knot forming step, the thread is wound on the second treatment tool two or more times.
